# EUROPEAN PATENT SPECIFICATION

(11) **EP 1 762 555 B1**
(45) Date of publication and mention of the grant of the patent: **27.05.2009**
(21) Application number: 05019593.2
(22) Date of filing: 08.09.2005
(51) Int. Cl.: C07C 29/151, C07C 31/04, C01B 3/50, B01J 8/00, B01J 19/00, B01D 53/00

(54) **Process for methanol production and related plant**
Verfahren zur Herstellung von Methanol und zugehörige Anlage
Procédé de préparation de méthanol et installation correspondante

(43) Date of publication of application: 14.03.2007
(73) Proprietor: METHANOL CASALE S.A., 6900 Lugano-Besso (CH)
(72) Inventor: Filippi, Ermanno, 6976 Castagnola (CH)
(74) Representative: Zardi, Marco

(56) References cited:
- EP-A- 0 849 245
- WO-A-20/05058783

## Description

### Field of application

In its most general aspect, the present invention concerns a process for methanol production from synthesis gas comprising carbon dioxide (CO2), carbon oxide (CO) and hydrogen (H2) obtained from natural gas reforming.

In particular, the present invention concerns a process for methanol synthesis of the type comprising the steps recited in the preamble of attached claim 1.

The present invention also concerns a plant for methanol production that carries out the aforementioned process.

As known, in the field of methanol production, there is an increasing need to make processes that are easy to carry out, which allow increasingly high production capacities to be reached with low operating and investment costs and low energy consumption.

### Prior art

It is well known that processes for methanol production involve reacting a gaseous mixture (or synthesis gas) comprising CO, CO2 and H2 in a synthesis section to obtain methanol, said gaseous mixture in turn being obtained from the natural gas reforming as defined above.

The reforming can be carried out in various ways but generally reforming processes in which the natural gas is reacted with steam, without the use of pure oxygen, to obtained the aforementioned gaseous mixture are very common, due to the relative simplicity and cost-effectiveness of making them. Such reforming processes are also known as primary reforming.

Although the processes for methanol production of the aforementioned type are advantageous from some points of view, they have the serious drawback that the gaseous mixture obtained from the primary reforming contains a substantial excess of H2 with respect to the stoichiometric amount required for conversion to methanol.

The excess of H2 in the gaseous mixture obtained from the primary reforming reflects negatively on the methanol synthesis section since it results in the need to make the corresponding apparatuses and instruments larger in size with consequent worsening of production costs. Moreover, the aforementioned excess of H2, acting as inert gas, also involves a smaller conversion to methanol of the gaseous mixture passing into the synthesis reactor with consequent worsening of manufacture and maintenance costs of such a reactor which must indeed be larger in size so as to be able to respect a predetermined production capacity. Last but not least, in this situation there is also greater energy consumption for methanol production.

It should also be noted that in processes of the aforementioned type, a gaseous flow comprising H2 in stoichiometric excess is purged from the synthesis section to then be used as a fuel in the primary reforming section with the prior separation of the methanol through washing of said gaseous flow.

Although on the one hand this allows the energy content of said gaseous flow to be at least partially recovered, on the other hand a part of the carbon content of the original gaseous synthesis mixture that can be converted into methanol and that comes out together with H2 from the synthesis section in the aforementioned purge gaseous flow in the form of CO, CO2 and some CH4, is irremediably lost.

It is thus clear that in this way the production capacity of the plant that implements such a methanol production process is reduced.

Precisely due to the cited drawbacks, the implementation of the processes for methanol production according to the prior art to this day requires great investment and high energy consumption such as to penalize the end production cost of the methanol.

EP-A-0 849 245 and WO 2005/058783 disclose a process for methanol production and related plant of the type recited in the preamble of attached claims 1 and 7.

The technical problem underlying the present invention is that of providing a process for methanol production that is simple to carry out and allows high production capacities to be obtained with low operating and investment costs, as well as with low energy consumption.

### Summary of the invention

Such a technical problem is solved by a process for methanol production of the type indicated above characterized in that it comprises the step recited in the characterizing portion of attached claim 1.

According to the invention, the aforementioned process for methanol production comprises the preliminary step of separating nitrogen from said gaseous flow comprising natural gas before feeding such a gaseous flow comprising natural gas to the primary reforming section.

This preliminary step is important since the natural gas used for the reforming generally also contains nitrogen in a significant quantity. In such a way, the nitrogen is prevented from being introduced into the methanol production plant, which would indeed cause a dilution effect of the reactant gases with a consequent reduction in the conversion yield into methanol.

By separating H2 from the purge gaseous flow so as to obtain a gaseous phase enriched with gaseous components containing carbon, in other words mainly CO and CO2, and recovering said gaseous phase for the primary reforming, a methanol production capacity is obtained in a simple way, with low operating and investment costs, and with low energy consumption.

In the present invention the gaseous components containing carbon of the purge gaseous flow, essentially CO and CO2, and CH4 originally contained in the initial natural gas and not converted in the primary reforming, are recovered for conversion into methanol instead of being used as a fuel.

Such gaseous components containing carbon of the purge gaseous flow are indeed recycled to the primary reforming section thus increasing the carbon content in the flow comprising natural gas to be subjected to primary reforming.

More specifically, in the primary reforming section, the CH4 contained in the purge gaseous flow is once again subjected to reforming with transformation thereof into CO and CO2 thus recovering its conversion potential into methanol, whereas CO and CO2 of the aforementioned purge gaseous flow are recovered for conversion into methanol. The CO and the CO2 of the purge gaseous flow also allow the yield of the primary reforming to be improved intervening in the reforming and shift reactions.

The overall result is therefore that in the process according to the invention, the gaseous synthesis mixture coming out from the primary reforming is rich in CO and CO2, in other words in the gases containing carbon to be used for the conversion into methanol, wherein nitrogen has been separated with respect to the known processes indicated above. This allows the production capacity of the methanol synthesis section to be increased.

The increased production capacity obtained with the process according to the invention also allows the size of the reactor, instruments and other apparatuses of the methanol synthesis section to be reduced - keeping the production the same - with this achieving low operating and investment costs, and low energy consumption.

It should also be noted that the process according to the invention does not exclude energy recovery of the excess hydrogen purged from the synthesis section, since the gaseous phase rich in H2 separated from the purge gaseous flow can also be recycled to the primary reforming section to be used as a fuel in the same way as occurs in the known processes indicated above.

In the process according to the invention, the separation of H2 from the purge gaseous flow is carried out by feeding said purge gaseous flow to a material that is semi-permeable to gas.

Preferably, the material that is semi-permeable to gas is selected between membranes and molecular sieves.

By the term "membranes" it is generally intended to mean conventional materials that are preferentially permeable to hydrogen.

By the term "molecular sieves", on the other hand, we generally means to include all conventional materials having micropores capable of preferentially absorbing CO, CO2 and CH4 contained in the gaseous mixture.

According to a preferred embodiment of the invention the recovery of said second gaseous phase comprising CO, CO2 and CH4 and substantially free of H2 for the reforming in said primary reforming section is carried out through the steps of:
- mixing said second gaseous phase comprising CO, CO2 and CH4 and substantially free of H2 with said gaseous flow comprising natural gas, and
- feeding the mixture resulting from said mixing to said primary reforming section.

Preferably, said separation of the nitrogen from the aforementioned gaseous flow comprising natural gas is carried out by feeding said gaseous flow comprising natural gas to a material that is semi-permeable to gas or through cryogenic separation.

Preferably, said material that is semi-permeable to gas is selected between membranes and molecular sieves.

The present invention also concerns a plant for methanol production as recited in attached claim 7.

The characteristics and advantages of the present invention are also clear from the following description of an embodiment thereof, given for indicating and not limiting purposes with reference to the attached figure.

### Brief description of the figure

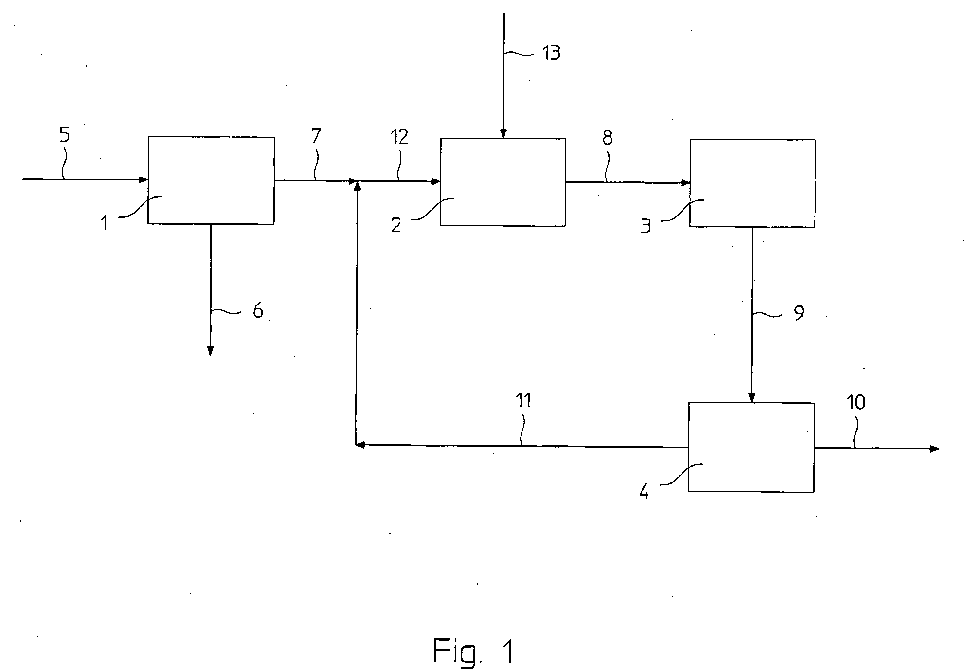

Figure 1 shows a block diagram of the process for methanol production according to the present invention.

### Detailed description

In figure 1, a block diagram is represented that illustrates the steps of the process according to the present invention for methanol production from a gaseous synthesis mixture comprising H2, CO and/or CO2 which is in turn obtained through primary reforming of a gaseous flow comprising natural gas. In the process illustrated hereafter, the raw material acting as the source of natural gas consists of natural gas.

With reference to the attached figure, block 1 indicates a unit for the separation of nitrogen from natural gas, block 2 indicates a primary reforming section, block 3 indicates a methanol synthesis section and block 4 indicates a unit for the separation of hydrogen from a purge gaseous flow.

The flow line 5 indicates a gaseous flow of natural gas containing a certain amount of nitrogen that enters into the nitrogen separation unit 1. The unit 1 comprises at least one membrane or a molecular sieve or appropriate cryogenic separation means, which are *per se* conventional for separating the nitrogen from the natural gas. At the outlet of the nitrogen separation unit 1, two gaseous flows are thus obtained and to be precise a gaseous flow (flow line 6) essentially consisting of nitrogen and a gaseous flow (flow line 7) of "purified" natural gas, in other words essentially free of nitrogen.

In the process illustrated here, which constitutes a preferred embodiment of the invention, the flow 7 of purified natural gas is mixed with a gaseous flow comprising CO, CO2 and CH4 and substantially free of H2 (flow line 11) coming from the H2 separation unit 4 (as shall be better explained hereafter). The resulting mixture is a gaseous flow that is fed into the primary reforming section 2 through the flow line 12. In the primary reforming section 2, the gaseous flow 12 is subjected to reforming in a conventional manner with steam, said steam being fed to the section 2 through the flow line 13. A gaseous mixture or synthesis gas comprising CO, CO2, H2 (the latter in stoichiometric excess) and CH4 is thus obtained.

The flow line 8 indicates a gaseous flow of the aforementioned gaseous synthesis mixture coming out from the primary reforming section 2, said gaseous flow being fed into the methanol synthesis section 3. In such a section 3, the gaseous mixture is reacted in an appropriate reactor in a *per se* conventional manner to obtain methanol.

Since, as stated previously, the gaseous synthesis mixture contains a substantial stoichiometric excess of H2, a gaseous flow comprising H2 and also CO, CO2 and CH4 is purged from the methanol synthesis section 3. Such a gaseous flow is indicated in figure 1 with the flow line 9.

The gaseous flow 9 is fed into the H2 separation unit 4, wherein H2 is separated from the other gaseous components of the gaseous flow 9. For this purpose the unit 4 comprises a *per se* conventional material that is semi-permeable to gas, preferably selected between membranes and molecular sieves.

At the outlet of the H2 separation unit 4 a gaseous flow (flow line 10) substantially consisting of H2 and the aforementioned gaseous flow 11 comprising CO, CO2 and CH4 and substantially free of H2 are therefore obtained.

The H2 contained in the gaseous flow 10 is then suitably released into the atmosphere or else advantageously recovered as an energy source, i.e. fuel, in the primary reforming section 2.

The gaseous flow 11 is recycled to the reforming section 2 so as to recover CO, CO2 and CH4 purged from the synthesis section 3 through the gaseous flow 9.

In the process illustrated here, such a gaseous flow 11 coming from the H2 separation unit 4 is mixed with the gaseous flow 7 of natural gas and the resulting mixture is fed to the primary reforming section 2.

The process illustrated above has the following main advantages:
- recovery of CO and CO2 of the purge gaseous flow 9 through the gaseous flow 11 with the possibility of converting them into methanol in the synthesis section 3. This means an increase in CO and CO2 in the synthesis reactor with consequent reduction in the stoichiometric excess of H2 and an increase in the methanol production capacity,
- recovery of CH4 purged from the synthesis section 3 with the gaseous flow 9 and conversion thereof into CO and CO2 in the primary reforming section 2,
- possibility of use of the gaseous flow 10 substantially comprising H2 as a fuel in the primary reforming section 2 for the purposes of reducing energy consumption.

The plant for methanol production according to the process of the present invention can be made ex-novo or else be obtained by a revamping (modernising) method of a pre-existing plant for methanol production.

In particular, in accordance with the preferred embodiment of the present invention represented in the example of figure 1, the plant for methanol production comprises a primary reforming section 2, for obtaining a gaseous mixture comprising CO, CO2, H2 in stoichiometric excess and CH4, connection means (flow lines 7, 13), for example *per se* conventional ducts, for feeding a gaseous flow comprising natural gas and a gaseous flow comprising steam to the primary reforming section 2, respectively, a methanol synthesis section 3, connection means (flow line 8), for example a *per se* conventional duct, for feeding the gaseous mixture comprising CO, CO2, H2 in stoichiometric excess and CH4 to the methanol synthesis section 3, purging means (for example a *per se* conventional control valve, not shown) for purging a gaseous flow comprising CO, CO2, H2 and CH4 from the synthesis section, a unit 4 for the separation of H2 from the purge gaseous flow, connection means (flow line 9), for example a *per se* conventional duct, for feeding the purge gaseous flow comprising CO, CO2, H2 and CH4 to the H2 separation unit 4, obtaining a first gaseous phase substantially consisting of H2 and a second gaseous phase comprising CO, CO2 and CH4 and substantially free of H2, recovery means (flow lines 11, 12) for the recovery of said second gaseous phase comprising CO, CO2 and CH4 and substantially free of H2 and sending it into said primary reforming section 2.

Preferably, the recovery means (11, 12) for the recovery and sending of said second gaseous phase comprising CO, CO2 and CH4 and substantially free of H2 to the primary reforming section 2 comprise:
- connection means (flow line 11), for example a *per se* conventional duct, for mixing said second gaseous phase comprising CO, CO2 and CH4 and substantially free of H2 with said gaseous flow comprising natural gas (flow line 7), and
- connection means (flow line 12), for example a *per se* conventional duct, for feeding the gaseous mixture resulting from the aforementioned mixing to the primary reforming section (2).

The plant for methanol synthesis according to the embodiment of figure 1 further comprises a unit 1 for the separation of nitrogen (N2) from the gaseous flow comprising natural gas before feeding such a gaseous flow to the primary reforming section 2.

The aforementioned plant for methanol production can be obtained through the modernisation of a pre-existing plant of the type comprising a primary reforming section 2 and a methanol synthesis section 3, thanks to the following operative steps: - providing a unit 4 for the separation of H2 from the purge gaseous flow comprising CO, CO2, H2 and CH4 coming out from the methanol synthesis section 3, - providing connection means (flow line 9), for example a *per se* conventional duct, for feeding the purge gaseous flow comprising CO, CO2, H2 and CH4 to the H2 separation unit 4, so as to obtain a first gaseous phase substantially consisting of H2 and a second gaseous phase comprising CO, CO2 and CH4 and substantially free of H2, and - providing recovery means (flow lines 11, 12) for the recovery of the second gaseous phase comprising CO, CO2 and CH4 and substantially free of H2 and for sending it into said primary reforming section 2.

The step of providing recovery means (11, 12) for the recovery of said second gaseous phase comprising CO, CO2 and CH4 and substantially free of H2 and for sending it into said primary reforming section (2) can comprise:
- providing connection means (flow line 11), for example a *per se* conventional duct, for mixing said second gaseous phase comprising CO, CO2 and CH4 and substantially free of H2 with said gaseous flow comprising natural gas (flow line 7), and
- providing connection means (flow line 12), for example a *per se* conventional duct, for feeding the gaseous mixture resulting from the aforementioned mixing to the primary reforming section (2).

### EXAMPLE

The characteristics of production capacity of a plant made according to the invention for methanol production are compared with those of a conventional plant that differs from the one according to the invention in that the purge gaseous flow is used as a fuel in the primary reforming section and in that it does not have a hydrogen separation unit.

The reforming and synthesis of methanol are carried out in the respective sections of the aforementioned plants in the operating conditions displayed in the table shown below, said table also displaying the results.

**TABLE**

| | Conventional plant | Plant according to the invention |
|---|---|---|
| Natural gas flow rate to the primary reforming section | 4600 kmol/h | 4600 kmol/h* *including the CH4 recycled from the synthesis section |
| Duty of the primary reforming | 100% | 100% |
| Flow rate of the gaseous mixture to the synthesis section | 17592 kmol/h | 18120 kmol/h |
| Stoichiometric ratio (H2-CO2 / CO+CO2) | 11.36 | 10.9 |
| Production | 2757 MTd* *:Million Ton per day | 2836 MTd* *:Million Ton per day |

From the results displayed in the aforementioned table it can clearly be seen that the plant for methanol production according to the invention has a lower stoichiometric ratio of H2 in the synthesis reactor and a higher production capacity than the conventional plant to which it is compared. In particular, the production capacity of the plant according to the invention is increased by 3% with respect to the conventional plant to which it is compared.

Of course, a man skilled in the art can bring numerous modifications and alternatives to the process and plant according to the invention, all of which are covered by the scope of protection of the following claims.

## Claims

1. Process for methanol synthesis comprising the steps of:
- feeding a gaseous flow comprising natural gas and a gaseous flow comprising steam to a primary reforming section,
- reacting the natural gas and the steam in the primary reforming section obtaining a gaseous mixture comprising CO, CO2, H2 in stoichiometric excess and CH4,
- feeding said gaseous mixture comprising CO, CO2, H2 in stoichiometric excess and CH4 to a methanol synthesis section and reacting it obtaining methanol,
- purging a gaseous flow comprising CO, CO2, H2 and CH4 from the synthesis section,
- separating H2 from said purge gaseous flow, obtaining a first gaseous phase substantially consisting of H2 and a second gaseous phase comprising CO, CO2 and CH4 and substantially free of H2,
- recovering said second gaseous phase comprising CO, CO2 and CH4 and substantially free of H2 for the reforming in said primary reforming section,
**characterized in that** it further comprises the preliminary step of separating N2 from said gaseous flow comprising natural gas before feeding said gaseous flow comprising natural gas to said primary reforming unit.

2. Process according to claim 1, **characterized in that** the recovery of said second gaseous phase comprising CO, CO2 and CH4 and substantially free of H2 for the reforming in said primary reforming section is carried out through the steps of:
- mixing said second gaseous phase comprising CO, CO2 and CH4 and substantially free of H2 with said gaseous flow comprising natural gas, and
- feeding the mixture resulting from said mixing to said primary reforming section.

3. Process according to claim 1 or 2, **characterized in that** said separation of H2 from said purge gaseous flow is carried out by feeding said purge gaseous flow to a material that is semi-permeable to gas.

4. Process according to claim 3, **characterized in that** said material that is semi-permeable to gas is selected between membranes and molecular sieves.

5. Process according to claim 1, **characterized in that** said separation of N2 from said gaseous flow is carried out by feeding said gaseous flow comprising natural gas to a material that is semi-permeable to gas or through cryogenic separation.

6. Process according to claim 5, **characterized in that** said material that is semi-permeable to gas is selected between membranes and molecular sieves.

7. Plant for methanol production comprising:
- a primary reforming section (2) for obtaining a gaseous mixture comprising CO, CO2, H2 in stoichiometric excess and CH4,
- connection means (7, 13) for feeding a gaseous flow comprising natural gas and a gaseous flow comprising steam to said primary reforming section (2), respectively,
- a synthesis section (3) for obtaining methanol,
- connection means (8) for feeding said gaseous mixture comprising CO, CO2, H2 in stoichiometric excess and CH4 to said methanol synthesis section (3),
- purging means for purging a gaseous flow comprising CO, CO2 H2 and CH4 from said methanol synthesis section (3),
- a unit (4) for the separation of H2 from said purge gaseous flow,
- connection means (9) for feeding said purge gaseous flow comprising CO, CO2, H2 and CH4 to said H2 separation unit (4), obtaining a first gaseous phase substantially consisting of H2 and a second gaseous phase comprising CO, CO2 and CH4 and substantially free of H2,
- recovery means (11, 12) of said second gaseous phase comprising CO, CO2 and CH4 and substantially free of H2 for the reforming in said primary reforming section (2),
**characterized in that** it further comprises:
- a unit (1) for the separation of N2 from said gaseous flow comprising natural gas before feeding said gaseous flow to said primary reforming section (2).

8. Plant for methanol production according to claim 7, **characterized in that** said recovery means (11, 12) of said second gaseous phase comprising CO, CO2 and CH4 and substantially free of H2 to said primary reforming section (2) comprise:
- connection means (11) for mixing said second gaseous phase comprising CO, CO2 and CH4 and substantially free of H2 with said gaseous flow comprising natural gas, and
- connection means (12) for feeding the gaseous mixture resulting from the aforementioned mixing to the primary reforming section (2).

## Patentansprüche

1. Prozess zur Methanolsynthese, folgende Schritte umfassend:
- Einleiten eines Erdgas enthaltenden Gasstroms und eines Dampf enthaltenden Gasstroms in einen primären Reformierabschnitt,
- das Erdgas und den Dampf im primären Reformierabschnitt zur Reaktion kommen zu lassen, wobei ein Gasgemisch gewonnen wird, das CO, CO₂, H₂ in stöchiometrischem Überschuss und CH₄ enthält,
- Einleiten des Gasgemischs, das CO, CO₂, H₂ in stöchiometrischem Überschuss und CH₄ enthält, in einen Methanolsyntheseabschnitt, und es zur Reaktion kommen zu lassen, wobei Methanol gewonnen wird,
- Abziehen eines Gasstroms, der CO, CO₂, H₂ und CH₄ enthält, aus dem Syntheseabschnitt,
- Abtrennen von H₂ von dem Abziehgasstrom, wobei eine erste Gasphase, die im Wesentlichen aus H₂ besteht, und eine zweite Gasphase gewonnen wird, die CO, CO₂ und CH₄ umfasst und im Wesentlichen frei von H₂ ist,
- Rückgewinnung der zweiten Gasphase, die CO, CO₂ und CH₄ enthält und im Wesentlichen frei von H₂ ist, zur Reformierung in dem primären Reformierabschnitt,
**dadurch gekennzeichnet, dass** er darüber hinaus den vorbereitenden Schritt umfasst, von dem Erdgas enthaltenden Gasstrom N₂ abzutrennen, bevor der Erdgas enthaltende Gasstrom in die primäre Reformiereinheit eingeleitet wird.

2. Prozess nach Anspruch 1, **dadurch gekennzeichnet, dass** die Rückgewinnung der zweiten Gasphase, die die CO, CO₂ und CH₄ enthält und im Wesentlichen frei von H₂ ist, und zur Reformierung in dem primären Reformierabschnitt vorgesehen ist, über folgende Schritte ausgeführt wird:
- Vermischen der zweiten Gasphase, die CO, CO₂ und CH₄ enthält und im Wesentlichen frei von H₂ ist, mit dem Erdgas enthaltenden Gasstrom, und
- Einleiten des sich aus dem Mischvorgang ergebenden Gemisches in den primären Reformierabschnitt.

3. Prozess nach Anspruch 1 oder 2, **dadurch gekennzeichnet, dass** die Abtrennung von H₂ vom Abziehgasstrom ausgeführt wird, indem der Abziehgasstrom in ein Material eingeleitet wird, das für Gas semipermeabel ist.

4. Prozess nach Anspruch 3, **dadurch gekennzeichnet, dass** das für Gas semipermeable Material aus Membranen und Molekularsieben ausgewählt ist.

5. Prozess nach Anspruch 1, **dadurch gekennzeichnet, dass** die Abtrennung von N₂ von dem Gasstrom ausgeführt wird, indem der Erdgas enthaltende Gasstrom in ein Material eingeleitet wird, das für Gas semipermeabel ist, oder durch eine Tieftemperaturabtrennung erfolgt.

6. Prozess nach Anspruch 5, **dadurch gekennzeichnet, dass** das für Gas semipermeable Material aus Membranen und Molekularsieben ausgewählt ist.

7. Anlage zur Methanolherstellung, umfassend:
- einen primären Reformierabschnitt (2), um ein Gasgemisch zu gewinnen, das CO, CO₂, H₂ in stöchiometrischem Überschuss und CH₄ enthält,
- Verbindungseinrichtungen (7, 13), um einen Erdgas enthaltenden Gasstrom und einen Dampf enthaltenden Gasstrom jeweils in den primären Reformierabschnitt (2) einzuleiten,
- einen Syntheseabschnitt (3) zur Gewinnung von Methanol,
- Verbindungseinrichtungen (8), um das Gasgemisch, das CO, CO₂, H₂ in stöchiometrischem Überschuss und CH₄ enthält, in den Methanolsyntheseabschnitt (3) einzuleiten,
- Abzieheinrichtungen, um einen Gasstrom, der CO, CO₂, H₂ und CH₄ enthält, von dem Methanolsyntheseabschnitt (3) abzuziehen;
- eine Einheit (4) zur Abtrennung von H₂ von dem Abziehgasstrom,
- Verbindungseinrichtungen (9), um den Abziehgasstrom, der CO, CO₂, H₂ und CH₄ enthält, in die H₂-Abtrenneinheit (4) einzuleiten, wobei eine erste Gasphase, die im Wesentlichen aus H₂ besteht, und eine zweite Gasphase gewonnen wird, die CO, CO₂ und CH₄ enthält und im Wesentlichen frei von H₂ ist,
- Rückgewinnungseinrichtungen (11, 12) der zweiten Gasphase, die CO, CO₂ und CH₄ enthält und im Wesentlichen frei von H₂ ist und zur Reformierung in dem primären Reformierabschnitt (2) vorgesehen ist,
**dadurch gekennzeichnet, dass** sie darüber hinaus umfasst:
- eine Einheit (1) zur Abtrennung von N₂ von dem Erdgas enthaltenden Gasstrom, bevor der Gasstrom in den primären Reformierabschnitt (2) eingeleitet wird.

8. Anlage zur Methanolherstellung nach Anspruch 7, **dadurch gekennzeichnet, dass** die Rückgewinnungseinrichtungen (11, 12) der zweiten Gasphase, die CO, CO₂ und CH₄ enthält und im Wesentlichen frei von H₂ ist und in den primären Reformierabschnitt (2) gelangt, Folgendes aufweisen:
- Verbindungseinrichtungen (11) zum Vermischen der zweiten Gasphase, die CO, CO₂ und CH₄ enthält und im Wesentlichen frei von H₂ ist, mit dem Erdgas enthaltenden Gasstrom, und
- Verbindungseinrichtungen (12) zum Einleiten des sich aus dem vorstehend erwähnten Mischvorgang ergebenden Gasgemisches in den primären Reformierabschnitt (2).

## Revendications

1. Procédé de synthèse de méthanol comportant les étapes consistant à :
- transférer un écoulement gazeux comportant du gaz naturel et un écoulement gazeux comportant de la vapeur, vers une section de reformage primaire,
- faire réagir le gaz naturel et la vapeur dans la section de reformage primaire en obtenant un mélange gazeux comprenant CO, CO₂ et H₂ en excès stoechiométrique et CH₄,
- transférer ledit mélange gazeux comportant CO, CO₂ et H₂ en excès stoechiométrique et CH₄, vers une section de synthèse de méthanol et le faire réagir en obtenant du méthanol,
- purger un écoulement gazeux comportant CO, CO₂, H₂ et CH₄ de la section de synthèse,
- séparer H₂ dudit flux gazeux de purge, en obtenant une première phase gazeuse sensiblement constituée de H₂ et une seconde phase gazeuse comportant CO, CO₂ et CH₄ et sensiblement exempte de H₂,
- récupérer ladite seconde phase gazeuse comportant CO, CO₂ et CH₄ et sensiblement exempte de H₂ pour le reformage dans ladite section de reformage primaire,
**caractérisé en ce qu'**il comporte en outre l'étape préliminaire consistant à séparer N₂ dudit écoulement gazeux comportant le gaz naturel avant de transférer ledit écoulement gazeux comportant le gaz naturel vers ladite section de reformage primaire.

2. Procédé selon la revendication 1, **caractérisé en ce que** la récupération de ladite seconde phase gazeuse comportant CO, CO₂ et CH₄ et sensiblement exempte de H₂ pour le reformage dans ladite section de reformage primaire est mise en oeuvre par les étapes consistant à :
- mélanger ladite seconde phase gazeuse comportant CO, CO₂ et CH₄ et sensiblement exempte de H₂ avec ledit écoulement gazeux comportant le gaz naturel, et
- transférer le mélange résultant dudit mélange vers ladite section de reformage primaire.

3. Procédé selon la revendication 1 ou 2, **caractérisé en ce que** ladite séparation du H₂ dudit écoulement gazeux de purge est effectuée en acheminant ledit écoulement gazeux de purge jusqu'à un matériau qui est semi-perméable au gaz.

4. Procédé selon la revendication 3, **caractérisé en ce que** ledit matériau qui est semi-perméable au gaz est choisi entre des membranes et des tamis moléculaires.

5. Procédé selon la revendication 1, **caractérisé en ce que** ladite séparation de N₂ dudit écoulement gazeux est effectuée en acheminant ledit écoulement gazeux comportant le gaz naturel jusqu'à un matériau qui est semi-perméable au gaz ou par séparation cryogénique.

6. Procédé selon la revendication 5, **caractérisé en ce que** ledit matériau qui est semi-perméable au gaz est choisi entre des membranes et des tamis moléculaires.

7. Installation de production de méthanol comportant :
- une section de reformage primaire (2) pour obtenir un mélange gazeux comportant CO, CO₂ et H₂ en excès stoechiométrique et CH₄,
- des moyens de liaison (7, 13) pour transférer un écoulement gazeux comportant du gaz naturel et un écoulement gazeux comportant de la vapeur, vers ladite section de reformage primaire (2), respectivement,
- une section de synthèse (3) pour obtenir du méthanol,
- des moyens de liaison (8) pour transférer ledit mélange gazeux comportant CO, CO₂ et H₂ en excès stoechiométrique et CH₄, vers ladite section de synthèse de méthanol (3),
- des moyens de purge pour purger un écoulement gazeux comportant CO, CO₂, H₂ et CH₄ à partir de la section de synthèse de méthanol (3),
- une unité (4) pour la séparation du H₂ dudit écoulement gazeux de purge,
- des moyens de liaison (9) pour transférer ledit écoulement gazeux de purge comportant CO, CO₂, H₂ et CH₄ vers ladite unité de séparation de H₂ (4), en obtenant une première phase gazeuse sensiblement constituée de H₂ et une seconde phase gazeuse comportant CO, CO₂ et CH₄ et sensiblement exempte de H₂,
- des moyens de récupération (11, 12) de ladite seconde phase gazeuse comportant CO, CO₂ et CH₄ et sensiblement exempte de H₂ pour le reformage dans ladite section de reformage primaire (2),
**caractérisée en ce qu'**elle comporte en outre :
une unité (1) pour la séparation de N₂ dudit écoulement gazeux comportant le gaz naturel avant de transférer ledit écoulement gazeux vers ladite section de reformage primaire (2).

8. Installation de production de méthanol selon la revendication 7, **caractérisée en ce que** lesdits moyens de récupération (11, 12) de ladite seconde phase gazeuse comportant CO, CO₂ et CH₄ et sensiblement exempte de H₂ jusqu'à ladite section de reformage primaire (2) comportent :
- des moyens de liaison (11) pour mélanger ladite seconde phase gazeuse comportant CO, CO₂ et CH₄ et sensiblement exempte de H₂ avec ledit écoulement gazeux comportant le gaz naturel, et
- des moyens de liaison (12) pour transférer le mélange gazeux résultant du mélange susmentionné vers la section de reformage primaire (2).
